# EUROPEAN PATENT APPLICATION

(11) **EP 2 954 778 A1**
(43) Date of publication of application: **16.12.2015**
(21) Application number: 14748526.2
(22) Date of filing: 06.02.2014
(51) Int. Cl.: A01K 67/027, G01N 33/15

(54) **ANIMAL MODEL FOR EVALUATING PERFORMANCE OF HEMOSTATIC AGENT FOR INDUCING HEMORRHAGE IN COMMON CAROTID ARTERY OR SUPERIOR SAGITTAL SINUS, AND USE THEREOF**

(30) Priority: 06.02.2013 KR 20130013557
(71) Applicant: InnoTherapy Inc., Seoul 152-741 (KR)
(72) Inventor: LEE, Moon Sue, Seoul 150-771 (KR); KOH, Mi Young, Incheon 403-010 (KR); KIM, Keumyeon, Seoul 138-864 (KR)
(74) Representative: Meissner, Bolte & Partner GbR
(86) International application number: PCT/KR2014/001034
(87) International publication number: WO 2014/123375

(57) **Abstract**

The present invention relates to an animal model for evaluating hemostatic performance, and the use thereof, and more particularly, to an animal model for evaluating the performance of a hemostatic agent, which has hemorrhage induced in the common carotid artery (CCA) or superior sagittal sinus (SSS) of the animal, a method of screening a hemostatic agent using the animal model, and a method of evaluating the effect of a hemostatic agent using the animal model. The animal model according to the present invention makes it possible to observe the hemostatic effect of a hemostatic agent in a rapid and accurate manner without causing side effects. Thus, the animal model is useful for screening a hemostatic agent and evaluating the effect of a hemostatic agent.

## Description

### TECHNICAL FIELD

The present invention relates to an animal model for evaluating hemostatic performance, and the use thereof, and more particularly, to an animal model for evaluating the21 performance of a hemostatic agent, which has hemorrhage induced in the common carotid artery (CCA) or superior sagittal sinus (SSS) of the animal, a method of screening a hemostatic agent using the animal model, and a method of evaluating the effect of a hemostatic agent using the animal model.

### BACKGROUND ART

As used herein, the term "medical instant adhesive" means, in a broad sense, medical supplies, including adhesive plasters, surgical adhesives and hemostatics, and in a narrow sense, adhesives that are used directly in medical fields, including dermatology, vascular surgery, gastroenterology and plastic surgery. Because the medical instant adhesive comes into contact with the skin, it should be biocompatible, should not be toxic and harmful to the body, should be biocompatible, and should have a hemostatic effect. In addition, it should show an instantaneous adhesive property even in the presence of moisture and should not interfere with the healing of the body.

Medical adhesive materials which are currently practically used include cyanoacrylates, fibrin glues, gelatin glues, and polyurethanes. Octyl cyanoacrylate which is a medical tissue adhesive (commercially available under the trade name "Dermabond" from Closure Medical Corp., USA) was approved for marketing by the EC in August, 1997 and approved for use by the US FDA in 1998. Ethicon, a subsidiary of Johnson & Johnson, has exclusively marketed this product in about 50 countries, including USA, Europe and Japan, and this product has been increasingly used worldwide for medical applications, including laceration healing, and the suture of incisions after plastic surgery and reconstructive surgery. In addition, there have been active studies on tissue adhesives, including 1,2-isopropylideneglyceryl 2-cyanoacrylates, alkyl 2-cyanoacryloyl glycolates, and methoxypropyl cyanoacrylates containing poly(trioxyethylene oxalate), in view of biocompatibility and biodegradability.

There have been studies focused on applying bioactive materials such as ligand peptides, which a proteinase substrate and a specific type of cell, to the adhesive material fibrin in order to impart functionality to the fibrin. Cohesion Corp. (USA) developed a fibrin-collagen composite tissue adhesive (CoStasis™), a hemostatic product containing thrombin and bovine collagen. The surgical glue "Tissel" was approved for use for heart bypass surgery, colorectal surgery, trauma and the like by the US FDA in 1998, and other several products are waiting for approval by the FDA.

In fact, animal model studies on medical adhesives, performed using test animals, have been reported as follows. Bijan S. caused damage to the aorta of rabbits to make animal models and measured the hemostatic effects of Gelfoam, Avitene, Surgicel and FloSeal, which are medical adhesives, by the use of the animal models (Bijan S et al., Journal of Surgical Research, 106:99-107, 2002). Moreover, Hasan Bilgili induced hemorrhage in the porcine skin, liver, spleen, vein and artery to make porcine hemorrhage models and evaluated hemostatic effects using the porcine hemorrhage models (Hasan Bilgili et al., Med Princ Pract, 18:165-169 2009). In addition, Ozer Kandemir et al. investigated the effects of new active hemostatic components by pathological and immunohistological analysis of rat models having aortic hemorrhage (Hasan Bilgili et al., Med Princ Pract, 18:165-169 2009).

However, in the case in which natural hemorrhage is difficult, like the case of blood gushing, an animal model that is used to demonstrate the hemostatic effects of hemostatic agents in a state in which blood gushes out has not been reported. In addition, in the case of the most severe hemorrhage such as arterial hemorrhage, that is, in the case in which blood gushes to a considerable height from a wound, blood flows out at high rate so that so that a large amount of blood will be lost. In this case, the blood is hardly coagulated, and thus it is not easy to evaluate the effect of a hemostatic agent.

In addition, in the preparation of a hemostatic animal model for evaluating the effect of a hemostatic agent, it is difficult to select blood vessels, due to the features of arteries and veins. For this reason, the preparation of the hemostatic animal model is limited.

Meanwhile, hemorrhage of the superior sagittal sinus is unavoidable and frequently occurs in sinus surgery. However, it is difficult to screen an effective hemostatic agent, because tissue surrounding the superior sagittal sinus is hard tissue. In addition, because an effective animal model for screening a composition for inhibiting hemorrhage does not exist, a hemostatic agent screened using soft tissue has been used.

Accordingly, the present inventors have made extensive efforts to develop a novel and effective animal model for evaluating hemostatic performance and a method of evaluating the effect of a hemostatic agent using the animal model, and as a result, have prepared an animal model having hemorrhage induced in the common carotid artery (CCA) or superior sagittal sinus (SSS) of the animal, and have found that the animal model is useful for testing the effect of a hemostatic agent, thereby completing the present invention.

### DISCLOSURE OF INVENTION

It is an object of the present invention to provide an animal model for evaluating the performance of a hemostatic agent.

Another object of the present invention is to provide a method of screening a hemostatic agent using the above animal model.

Still another object of the present invention is to provide a method of evaluating the effect of a hemostatic agent using the above animal model.

To achieve the above objects, the present invention provides an animal model for evaluating the effect of a hemostatic agent, which has hemorrhage induced in the common carotid artery (CCA) or superior sagittal sinus (SSS) thereof.

The present invention also provides a method of screening a hemostatic agent from candidate hemostatic agents by the use of the above animal model for evaluating the effect of a hemostatic agent.

The present invention also provides a method of evaluating the effect of a hemostatic agent by the use of the above animal model for evaluating the effect of a hemostatic agent.

### BRIEF DESCRIPTION OF THE DRAWINGS

FIG. 1 shows a step of anesthetizing a test animal.
FIG. 2 shows a test animal whose head has been fixed on a stereotaxic frame after anesthesia and the shaving of the site to be surgically operated.
FIG. 3 shows a step of applying a lubricant to a temperature sensor, lifting up the tail of a test animal, and then inserting the temperature into the anus.
FIG. 4 shows incising the surgical site of an animal model and removing the connective tissue to expose the arteries.
FIG. 5 shows the common carotid artery (CCA) that is the surgical site of an arterial model.
FIG. 6 shows a hemostatic agent applied to cotton for hemostasis after the induction of hemorrhage.
FIG. 7 shows incising the scalp of a vein model.
FIG. 8 shows a surgical site ensured by incision of the cranium and the position of the superior sagittal sinus (SSS).
FIG. 9 shows the results of testing the hemostatic effects of candidate hemostatic agents using an artery animal model.
FIG. 10 shows the results of testing the hemostatic effects of candidate hemostatic agents using a vein animal model.

Other features and embodiments of the present invention will be more apparent from the following detailed descriptions and the appended claims.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods, which will be described below, are those well known and commonly employed in the art.

In one aspect, the present invention is directed to an animal model for evaluating the effect of a hemostatic agent, which has hemorrhage induced in the common carotid artery (CCA) or superior sagittal sinus (SSS) thereof.

In an embodiment, an animal model for evaluating the effect of a hemostatic agent according to the present invention may be prepared by a method comprising the steps of: (a) ensuring an arterial surgical site in an animal; and (b) inducing hemorrhage in the common carotid artery (CCA) of the animal.

In another embodiment, an animal model for evaluating the effect of a hemostatic agent according to the present invention may be prepared by a method comprising the steps of: (a') ensuring a venous surgical site in an animal; and (b') inducing hemorrhage in the superior sagittal sinus (SSS) of the animal.

In the present invention, the method may further comprise, before step (a), a step of anesthetizing the animal.

Anesthesia of the animal may be performed according to any conventional method known to those skilled in the art. An anesthesia method that is used to anesthetize the animal in the present invention is preferably a method of anesthetizing the animal with Zoletil/Rompun; a method of anesthetizing the animal with ketamine/xylazine/acepromazine; a method of anesthetizing the animal with sodium pentobarbital alone; or a method of anesthetizing the animal with isoflurane, but is not specifically limited thereto.

In an embodiment of the present invention, anesthesia may be performed using Zoletil/Rompun, a fluid (lactated Ringer's solution, etc.) may be administered to the animal during a surgical operation, and an antibiotic and an analgesic may be administered after the surgical operation.

The animal that is used in steps (a) and (a') may include all animals in which hemorrhage can be induced. Preferably, it may include mammals. More preferably, it may include rodents such as mice, guinea pigs, hamsters and rats, and non-human Primates such as cats, dogs, pigs, rabbits, sheep, goats, deer, horses, cattle, mandrills, chimpanzees and monkeys.

The common carotid artery (CCA) and superior sagittal sinus (SSS) that are used in steps (b) and (b') are characterized by having site-specific characteristics having no concern with the animal.

For example, the animal used may be a 7-15-week-old rodent. Preferably, the animal is a rat. More preferably, the animal may be a 10-week-old individual (weight: 350-500 g).

In the present invention, an artery has a large amount of hemorrhage, and thus is used as a standard for quantifying the hemorrhage time and site. Also, a vein is used as a standard for normalizing cranial incision and selecting a vein.

In the present invention, the common carotid artery (CCA) is a carotid artery that supplies blood from the heart to the brain and the heart, and has a characteristic in that it gushes out blood when being damaged. Thus, when hemorrhage is induced in the common carotid artery (CCA), an effective animal model can be prepared, which enables the testing of the effect of a hemostatic agent.

Hereinafter, the step of preparing an animal model having hemorrhage induced in the common carotid artery (CCA) will be described in detail.

In the present invention, the animal is anesthetized and fixed, after which the surgical site is shaved and disinfected with betadine. Then, the midline of a site ranging from the chest line to the neck of the animal is preferably incised by about 1-5 cm by the use of scissors, followed by removal of connective tissues with cotton swabs. More preferably, the midline of the site ranging from the chest line to the neck is incised by about 3-4 cm.

Omohyoid muscle extending above the blood vessel of the incised site is cut with cotton swabs, and connective tissue around the blood vessels is removed with forceps under microscopic observation to ensure the common carotid artery (CCA). Preferably, a sufficient space is ensured above and below the common carotid artery (CCA) in order to facilitate compression.

The ensured common carotid artery (CCA) is damaged by pricking it with a 10-40G needle at a certain angle, thereby inducing hemorrhage. Preferably, the common carotid artery (CCA) is pricked with a 31G syringe needle at an angle of about 30° to induce hemorrhage.

When the superior sagittal sinus (SSS) is damaged, natural hemorrhage is difficult, and side effects such as obstruction are likely to occur. Thus, according to the present invention, hemorrhage is induced in the superior sagittal sinus (SSS). In this case, an effective animal model can be prepared, which enables the testing of the effect of a hemostatic agent.

Hereinafter, the step of preparing an animal model having hemorrhage in the superior sagittal sinus (SSS) will be described in detail.

First, the midline of a site ranging from the middle of the forehead to the ear portion is incised by about 1-5 cm (preferably about 2-3 cm) using a scalpel (No. 10). The incised portion is opened and fixed with forceps, and connective tissues are removed with cotton swabs, after which the cranium is exposed.

Next, the middle of the Lambda and Bregma of the exposed cranium is ground by about 1-10 mm using a grinder. Preferably, it is ground by about 5 mm using a diamond grinder. Next, the bone is lightly exposed using wither forceps having a sharp tip or a scalpel.

After removal of the cranium, the exposed vein is damaged using a 10-40G medicut to induce hemorrhage.

The animal model having hemorrhage induced in the common carotid artery (CCA) or the superior sagittal sinus (SSS) according to the present invention may be used to evaluate the effects of candidate hemostatic agents or to screen a hemostatic agent from candidate hemostatic agents.

After the induction of hemorrhage, the hemorrhage-induced site of the common carotid artery (CCA) is compressed with cotton, or covered with cotton having a candidate hemostatic agent applied thereto and compressed for 1-10 minutes. Next, the cotton is removed, and whether hemostasis has been achieved is examined. Preferably, cotton having a candidate hemostatic agent (0.5 ml) applied thereto is applied to the hemorrhage site which is then compressed, and after 2 minutes, whether hemostasis has been achieved is examined.

After the induction of hemorrhage, 0.1-1 ml of a candidate hemostatic agent is preferably applied to the hemorrhage-induced site of the superior sagittal sinus (SSS), and after 2 minutes, whether hemostasis has been achieved is examined.

In another aspect, the present invention is directed to a method of screening a hemostatic agent from candidate hemostatic agents by the use of an animal model for evaluating the effect of a hemostatic agent.

In still another aspect, the present invention is directed to a method of evaluating the effect of a hemostatic agent by the use of an animal model for evaluating the effect of a hemostatic agent.

In the present invention, the candidate hemostatic agent is applied to the surgical site of the animal model after it is suspended in a solvent.

### EXAMPLES

Hereinafter, the present invention will be described in further detail with reference to examples. It will be obvious to a person having ordinary skill in the art that these examples are illustrative purposes only and are not to be construed to limit the scope of the present invention. Thus, the substantial scope of the present invention will be defined by the appended claims and equivalents thereof.

### Example 1: Preparation of animal model (artery) for evaluating hemostatic effects

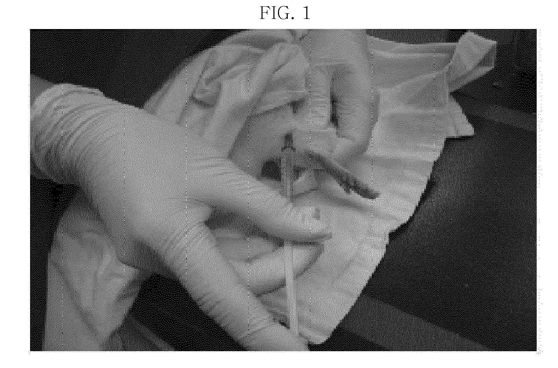

To prepare an animal model for evaluating hemostatic effects, the hind leg muscle of a rat was anesthetized by intramuscular injection of 0.1 ml/100 g of Zoletil/Rompun (5 ml Zoletil + 2.5 ml Rompun), and after 20 minutes, a surgical operation for the rat was started (FIG. 1).

The animals used in this Example were 10-week-old SD rats (weight: about 350-450 g).

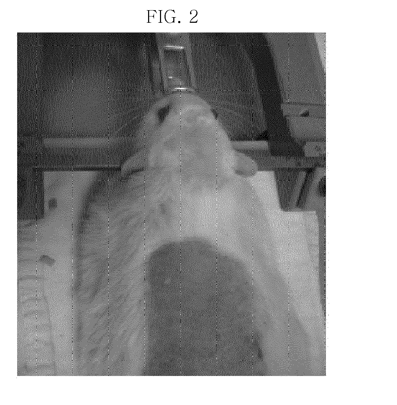

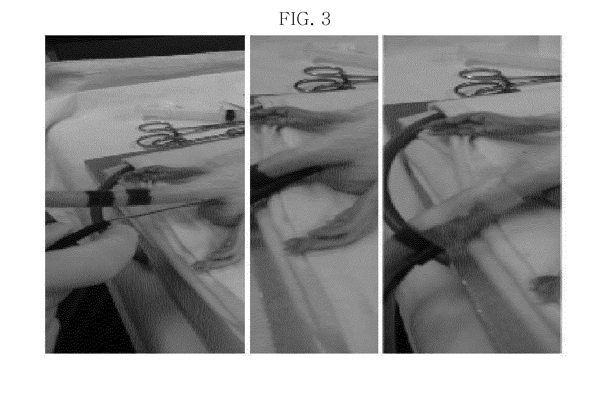

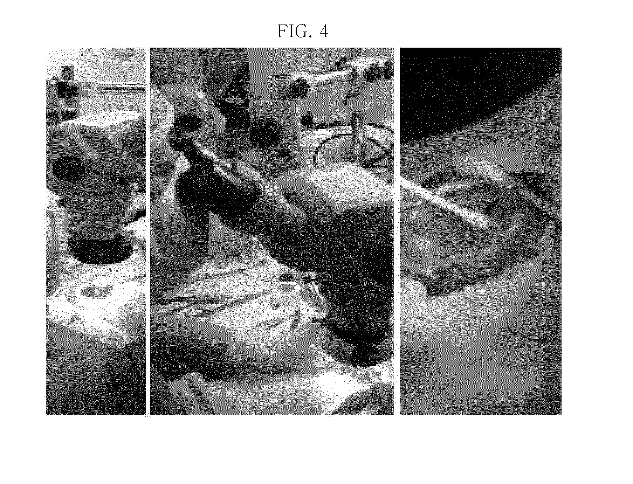

In addition, the site to be surgically operated was widely shaved, and the rat was fixed on a stereotaxic frame (FIG. 2). To maintain the body temperature at a constant level, a body temperature sensor with a warm pad was inserted into the anus of the rat and fixed (FIG. 3). The shaved portion of the animal was disinfected with betadine, and then the midline of a site ranging from the chest line to the neck portion was incised by about 3-4 cm. Next, as shown in FIG. 4, connective tissues were removed with cotton swabs, and omohyoid muscle extending above the blood vessel was cut with cotton swabs (FIG. 4).

In addition, while the surgical site was observed with a microscope, connective tissues around the blood vessel were removed with forceps, thereby ensuring a sufficient space above and below the common carotid artery (CCA) to facilitate compression.

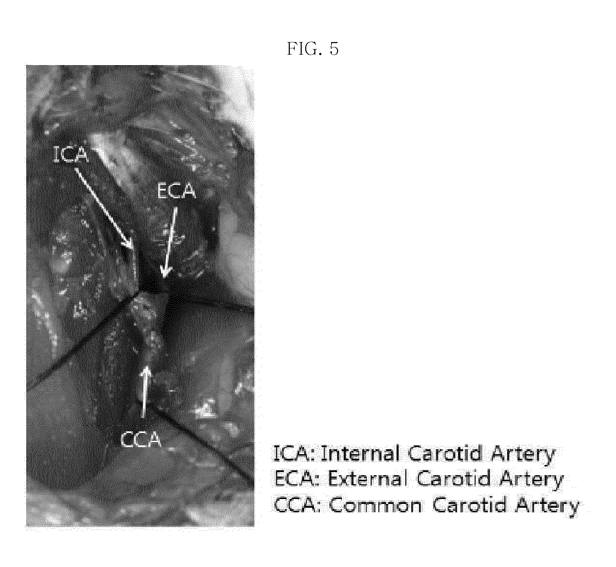

Hemorrhage in the ensured blood vessel was induced by pricking the blood vessel with a 31G syringe needle at an angle of about 30° in such a manner that the inclined portion of the needle faced upward, thereby preparing an animal model for evaluating hemostatic effects (FIG. 5).

### Example 2: Preparation of animal model (vein) for evaluating hemostatic effects

To prepare an animal model for evaluating hemostatic effects, the hind leg muscle of a rat was anesthetized by intramuscular injection of 0.1 ml/100 g of Zoletil/Rompun (5 ml Zoletil + 2.5 ml Rompun), and after 20 minutes, a surgical operation for the rat was started (FIG. 1).

The animals used in this Example were 10-week-old SD rats (weight: about 350-450 g).

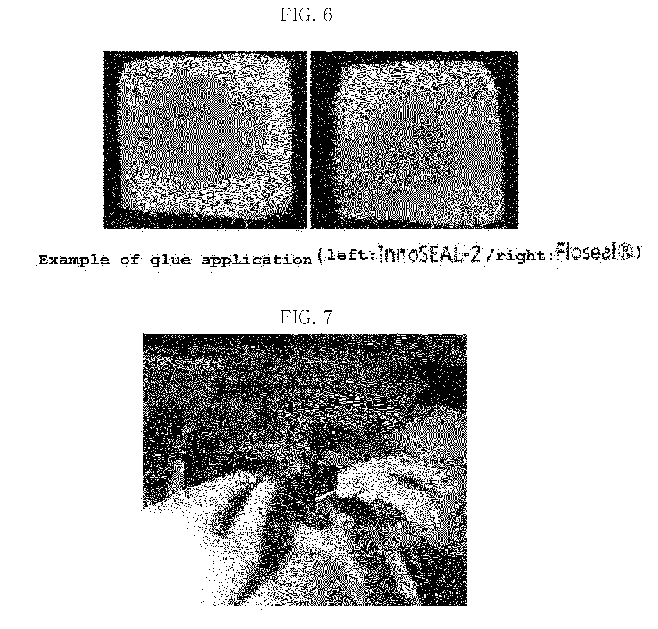

In addition, the site to be surgically operated was widely shaved, and the rat was fixed on a stereotaxic frame (FIG. 2). To maintain the body temperature at a constant level, a body temperature sensor with a warm pad was inserted into the anus of the rat and fixed (FIG. 3). The shaved portion of the animal was disinfected with betadine, and then the midline of a site ranging from the middle of the forehead to the ear portion was incised by about 2-3 cm using a scalpel (No. 10). The incised portion was opened and fixed with forceps, and connective tissues were removed with cotton swabs, after which the cranium was exposed (FIG. 7).

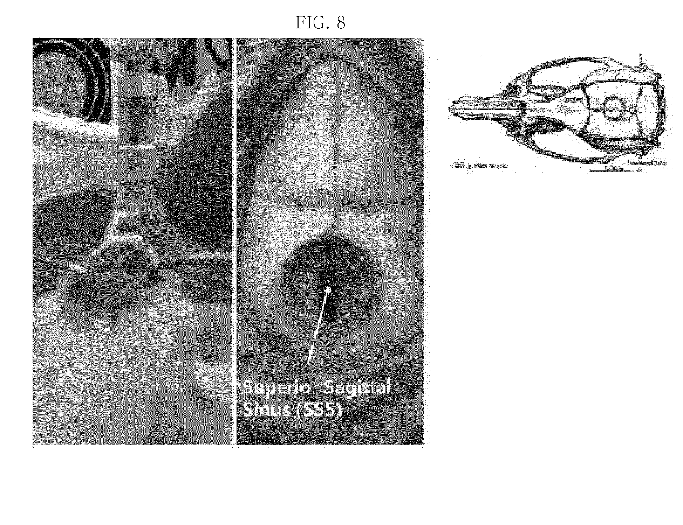

As shown in FIG. 8, the middle of the Lambda and Bregma of the cranium was ground by about 5 mm using a diamond grinder. Next, the middle was lightly ground using either forceps having a sharp tip or a scalpel so that the bone would be exposed. Next, hemorrhage in the superior sagittal sinus (SSS) (middle of Lambda and Bregma) was induced using a 20G medicut (whose inclined portion faced upward), thereby preparing an animal model for evaluating hemostatic effects.

### Example 3: Preparation of animal model (liver) for evaluating hemostatic effects

To prepare an animal model for evaluating hemostatic effects, the hind leg muscle of a rabbit was anesthetized by subcutaneous injection of 0.5 ml/kg of Zoletil/Rompun (5 ml Zoletil + 2.5 ml Rompun), and after 20 minutes, a surgical operation for the rabbit was started.

The animals used in this Example were New Zealand White rabbits (weight: about 2.3-3.0 kg).

In addition, the site to be surgically operated was widely shaved and locally anesthetized with lidocaine, and the animal was fixed on a surgical operation table equipped with hyperhypo thermia.

The thorax was opened by incision, and then the liver central lobe was exposed onto a gauze wet with sterile physiological saline. The middle portion of the liver (central lobe) was pierced to a depth of about 2-3 mm using a biopsy punch (d=6 mm) to induce hemorrhage, thereby preparing an animal model.

### Example 4: Preparation of animal model (femoral artery) for evaluating hemostatic effects

To prepare an animal model for evaluating hemostatic effects, the hind leg muscle of a rabbit was anesthetized by subcutaneous injection of 0.5 ml/kg of Zoletil/Rompun (5 ml Zoletil + 2.5 ml Rompun), and after 20 minutes, a surgical operation for the rabbit was started.

The animals used in this Example were New Zealand White rabbits (weight: about 2.3-3.0 kg).

In addition, the site to be surgically operated was widely shaved, and the animal was fixed on a surgical operation table equipped with hyperhypo thermia. 0.4 mL of undiluted heparin sodium (JW Pharmaceutical Corp., Korea; 25,000 I.U./5 mL) was mixed with 3.6 mL of water for injection (JW Pharmaceutical Corp.), and 0.4 mL of the solution was injected into the animal, after which the surgical site was disinfected with betadine. The skin of the surgical site was incised within 15 minutes, after which the fascia and muscle were incised to expose the femoral artery. After 15 minutes, the exposed femoral artery was pricked with a 23G needle to induce hemorrhage, thereby preparing an animal model.

### Example 5: Evaluation of hemostatic effects using animal model having hemorrhage induced in common carotid artery (CCA)

0.5 ml of a candidate hemostatic agent suspended in a suitable solvent was applied to the hemorrhage-induced site of the animal model having hemorrhage induced in the common carotid artery (CCA), prepared in Example 1 (FIG. 6).

The hemorrhage-induced site was compressed either with cotton or with cotton having a hemostatic agent applied thereto. During the compression, the hemorrhage-induced site was compressed laterally so that the airway would not be pressed.

The hemorrhage-induced site was compressed while whether the animal would breathe was checked. After 2 minutes, the cotton was removed, and whether hemostasis was achieved was examined. Herein, the cotton was carefully removed so as not to influence clot.

In this Example, the animal models prepared in Example 1 were divided into four groups, each consisting of 5 animals, and the hemostatic effects of InnoSEAL™⁻² and InnoSEAL™⁻³ that are candidate hemostatic agents were evaluated using the animal models.

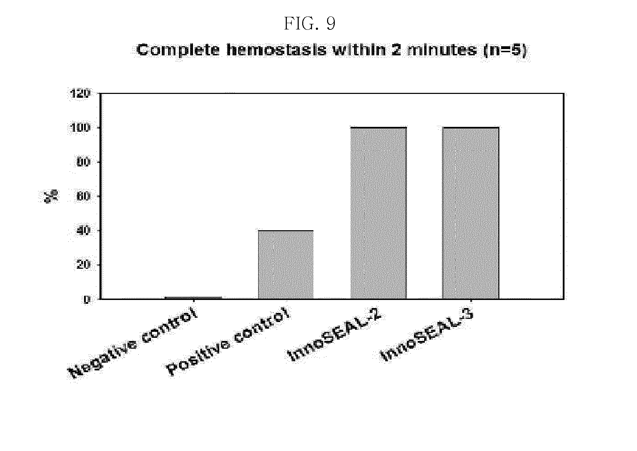

As a result, as shown in FIG. 9, InnoSEAL™⁻² and InnoSEAL™⁻³ showed excellent hemostatic effects compared to commercially available Floseal (positive control). In FIG. 9, the negative control is a group not treated with anything.

For individual animals for evaluating the stability of the animal models, the individual animals were monitored for 2 weeks to observe the survival of the animals and a change in the weight of the animals.

### Example 6: Evaluation of hemostatic effects using animal model having hemorrhage induced in superior sagittal sinus (SSS)

0.1 ml of a candidate hemostatic agent suspended in a suitable solvent was applied to the hemorrhage-induced site of the animal model having hemorrhage induced in the superior sagittal sinus, prepared in Example 2.

After the application of the candidate hemostatic agent, the blood that did flow 2 minutes after induction of hemorrhage was washed out, and whether hemostasis was achieved was examined. After completion of the test, the skin was sutured with 4-0 polyamide threads and disinfected with betadine, and 0.1 ml of an antibiotic (Cefamezin injection) was administered thereto.

In this Example, the animal models prepared in Example 1 were divided into four groups, each consisting of 4 animals, and the hemostatic effects of InnoSEAL™⁻² and InnoSEAL™⁻³ that are candidate hemostatic agents were evaluated using the animal models.

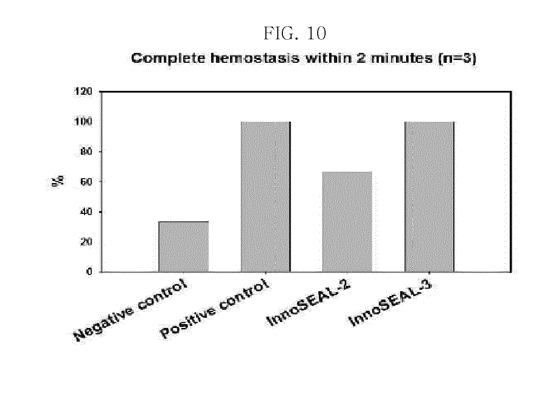

As a result, as shown in FIG. 10, InnoSEAL™⁻² and InnoSEAL™⁻³ showed excellent hemostatic effects compared to commercially available Floseal (positive control). In FIG. 10, the negative control is a group not treated with anything. The individual animals were monitored for 2 weeks to observe the survival of the animals and a change in the weight of the animals. After completion of the test, the animals were euthanized.

Individual animals for evaluating the stability of the animal models were sacrificed by perfusion at days 3, 7, 14 and 28, after which the brains were exposed. Then, the portions that came into contact with the hemostatic agent were histologically observed.

### Example 7: Evaluation of hemostatic effects using animal model having hemorrhage induced in liver tissue (central lobe)

A candidate hemostatic agent suspended in a suitable solvent was applied to the hemorrhage-induced site of the animal model having hemorrhage induced in the liver tissue, prepared in Example 3. The hemorrhage inhibitory composition was applied at 10 seconds after hemorrhage, and within 4 minutes, whether hemorrhage was achieved was visually evaluated. Group 1 was a negative control not treated with anything, and group 2 was a positive control to which Floseal^{®} (Baxter) was applied so as to completely cover the hemorrhage site. To group 3, InnoSEAL hydrogel was applied so as to completely cover the hemorrhage site. In the case of groups 2 and 3, when the amount of hemorrhage was large, the hemorrhage inhibitory composition was applied in an amount of up to 4 ml. In the case of group 4, one or two sheets of InnoSEAL sponge were placed on the liver so as to completely cover the hemorrhage site. InnoSEAL hydrogel was removed after 4 minutes, and InnoSEAL sponge was removed after 4 minutes, after which whether hemorrhage was achieved was evaluated.

After completion of the evaluation, the surgical site of each rabbit was sutured, and 0.5 mL Meloxicam (analgesic; Meloxicam, Boehringer Ingelheim) and 0.4 mL Cefazolin sodium (antibiotic; Chong Kun Dang Pharmaceutical Corp., Korea) were injected into each rabbit, after which the survival of the rabbits was checked for 24 hours.

As a result, as can be seen in Table 1 below, InnoSEAL hydrogel and InnoSEAL sponge all had excellent hemostatic effects compared to that of commercially available Floseal^{®}.

Particularly, InnoSEAL sponge showed a hemostasis achievement rate of 100% within 4 minutes, suggesting that it very effectively inhibits hemorrhage of liver tissue.

**Table 1**

| **Groups** | **Individual No.** | **Hemostatic time** | **Hemostasis achievement ratio within 4 min.** |
|---|---|---|---|
| Group1 | 1 | 7 min. 30 sec. | 0% |
| | 2 | 8 min. 30 sec. | |
| | 3 | 7 min. 00 sec. | |
| | 4 | More than 10 min | |
| | 5 | 6 min. 30 sec. | |
| Group2 | 1 | 5 min. 00 sec. | 40% |
| | 2 | 3 min. 00 sec. | |
| | 3 | 5 min. 00 sec. | |
| | 4 | 5 min. 00 sec. | |
| | 5 | 3 min. 00 sec. | |
| Group3 | 1 | 5 min. 00 sec. | 60% |
| | 2 | More than 10 min | |
| | 3 | 4 min. 00 sec. | |
| | 4 | 4 min. 00 sec. | |
| | 5 | 4 min. 00 sec. | |
| Group4 | 1 | 3 min. 00 sec. | 100% |
| | 2 | 4 min. 00 sec. | |
| | 3 | 4 min. 00 sec. | |
| | 4 | 3 min. 00 sec. | |
| | 5 | 3 min. 00 sec. | |

### Example 8: Evaluation of hemostatic effects using animal model having hemorrhage induced in femoral artery

A candidate hemostatic agent suspended in a suitable solvent was applied to the hemorrhage-induced site of the animal model having hemorrhage induced in the liver tissue, prepared in Example 4. Group 1 was a negative control in which the hemorrhage site was covered with cotton not treated with anything and was compressed to induce hemostasis, and group 2 is a positive control in which the hemorrhage site was covered with cotton having 1 mL of Floseal^{®} (Baxter) applied thereto and was compressed to induce hemostasis. In the case of Group 3, the hemorrhage site was covered with cotton having 1 mL of InnoSEAL hydrogel applied thereto and was compressed to induce hemostasis. In the case of group 4, the hemorrhage site was covered with cotton having two InnoSEAL sponge sheets placed thereon and was compressed to induce hemostasis.

At 1 minute and 30 seconds, 2 minutes and 30 seconds, 3 minutes and 30 seconds, 5 minutes and 7 minutes after induction of hemostasis, the cotton was carefully removed, and whether hemostasis was achieved was visually evaluated. After completion of the evaluation, the test animals were injected intramuscularly with an analgesic (Meloxicam) and an antibiotic (Cefamezin, Cefazolin sodium), and the survival of the animals was checked for 24 hours.

As a result, as can be seen in Table 2 below, commercially available Floseal^{®} did not achieve hemostasis within 1 minute and 30 seconds, whereas InnoSEAL showed hemostatic effects within 1 minute and 30 seconds. Particularly, it was shown that InnoSEAL sponge showed a hemostasis achievement rate of 80%, indicating that it showed a significantly excellent hemostatic effect in the femoral artery compared to other materials. In addition, InnoSEAL sponge showed a mean hemostasis time of 1 minutes and 42 seconds, which is very short.

**Table 2**

| **Groups** | **Individual No.** | **Hemostatic time** | **Hemostasis achievement ratio within 1 min. 30 sec.** |
|---|---|---|---|
| Group1 | 1 | 7 min. 00 sec. | 0% |
| | 2 | 5 min. 00 sec. | |
| | 3 | 7 min. 00 sec. | |
| | 4 | 7 min. 00 sec. | |
| | 5 | 5 min. 00 sec. | |
| Group2 | 1 | 2 min. 30 sec. | 0% |
| | 2 | 2 min. 30 sec. | |
| | 3 | 2 min. 30 sec. | |
| | 4 | 3 min. 30 sec. | |
| | 5 | 2 min. 30 sec. | |
| Group3 | 1 | 2 min. 30 sec. | 20% |
| | 2 | 1 min. 30 sec. | |
| | 3 | 3 min. 30 sec. | |
| | 4 | 2 min. 30 sec. | |
| | 5 | 2 min. 30 sec. | |
| Group4 | 1 | 1 min. 30 sec. | 80% |
| | 2 | 2 min. 30 sec. | |
| | 3 | 1 min. 30 sec. | |
| | 4 | 1 min. 30 sec. | |
| | 5 | 1 min. 30 sec. | |

### INDUSTRIAL APPLICABILITY

As described above, the animal model according to the present invention makes it possible to observe the effects of hemostatic agents in a rapid and accurate manner without causing side effects. Thus, the animal model is useful for screening hemostatic agents and evaluating the effects of hemostatic agents.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. An animal model for evaluating the effect of a hemostatic agent, which has hemorrhage induced in the common carotid artery (CCA) thereof.

2. The animal model of claim 1, wherein the animal model for evaluating the effect of a hemostatic agent is prepared by a method comprising the steps of: (a) ensuring an arterial surgical site in an animal; and (b) inducing hemorrhage in the common carotid artery (CCA) of the animal.

3. The animal model of claim 2, wherein the animal a rat.

4. The animal model of claim 2, wherein in step (b), connective tissue around the blood vessels is removed to ensure the arterial surgical site in an animal, and then is hemorrhage is induced in the common carotid artery (CCA) using a syringe.

5. An animal model for evaluating the effect of a hemostatic agent, which has hemorrhage induced in the superior sagittal sinus (SSS) thereof.

6. The animal model of claim 5, wherein the animal model for evaluating the effect of a hemostatic agent is prepared by a method comprising the steps of: (a) ensuring a venous surgical site in an animal; and (b) inducing hemorrhage in the superior sagittal sinus (SSS) of the animal.

7. The animal model of claim 6, wherein the animal a rat.

8. The animal model of claim 6, wherein in step (b), the superior sagittal sinus (SSS) is damaged using a 20G medicut to induce hemorrhage in the superior sagittal sinus (SSS).

9. A method of screening a hemostatic agent from candidate hemostatic agents by the use of the animal model of any one of claims 1 to 4.

10. The method of claim 9, wherein a candidate hemostatic agent is applied to the hemorrhage-induced site of the animal model which is in turn compressed, and then whether hemostasis has been achieved is examined.

11. A method of screening a hemostatic agent from candidate hemostatic agents by the use of the animal model of any one of claims 5 to 8.

12. The method of claim 11, wherein a candidate hemostatic agent is applied to the hemorrhage-induced site of the animal model which is in turn compressed, and then whether hemostasis has been achieved is examined.

13. A method of evaluating the effect of a hemostatic agent by the use of the animal model of any one of claims 1 to 8.
